## Europäisches Patentamt
## European Patent Office
## Office européen des brevets

(19)

(11) Veröffentlichungsnummer: **0 197 149**
**B1**

(12) **EUROPÄISCHE PATENTSCHRIFT**

(45) Veröffentlichungstag der Patentschrift: **02.05.90**

(51) Int. Cl.⁵: **A 61 K 37/66,** A 61 K 35/14, C 07 K 15/26

(21) Anmeldenummer: **84904228.8**

(22) Anmeldetag: **21.09.84**

(86) Internationale Anmeldenummer:
**PCT/SU84/00051**

(87) Internationale Veröffentlichungsnummer:
**WO 86/01722 27.03.86 Gazette 86/07**

(54) **VERFAHREN ZUR GEWINNUNG MENSCHLICHEN LEUKOZYTEN-INTERFERONS.**

(43) Veröffentlichungstag der Anmeldung:
**15.10.86 Patentblatt 86/42**

(45) Bekanntmachung des Hinweises auf die
Patenterteilung: **02.05.90 Patentblatt 90/18**

(84) Benannte Vertragsstaaten:
**BE CH DE FR LI SE**

(56) Entgegenhaltungen:
**WO-A-/01899**
**FR-A-2 457 103**
**SU-A- 152 543**

(73) Patentinhaber: **VSESOJUZNY NAUCHNO-ISSLEDOVATELSKY INSTITUT OSOBO CHISTYKH BIOPREPARATOV**
**ul. Pudozhskaya 7**
**Leningrad, 197011 (US)**

(72) Erfinder: **KOROBITSYN, Leonid Pavlovich**
**ul. Kompozitorov, 11/1-8**
**Leningrad, 194355 (SU)**
Erfinder: **PIVOVAROV, Anatoly Mikhailovich**
**pr. K. Marxa, 7-15a**
**Leningrad, 194175 (SU)**
Erfinder: **GONCHAROVA, Svetlana Viktorovna**
**ul. Divenskaya, 18-17**
**Leningrad, 197061 (SU)**
Erfinder: **KULIKOVA, Irina Nikolaevna**
**pr. Maklina, 26-14**
**Leningrad, 190121 (SU)**
Erfinder: **TYAGOTIN, Jury Vasilievich**
**pr. Udarnikov, 21-1-244**
**Leningrad, 195279 (SU)**

(74) Vertreter: **von Füner, Alexander, Dr. et al**
**Patentanwälte v. Füner, Ebbinghaus, Finck**
**Mariahilfplatz 2 & 3**
**D-8000 München 90 (DE)**

Courier Press, Leamington Spa, England.

EP 0 197 149 B1

**Beschreibung**

Die Erfindung bezieht sich auf die industrielle Biologie, insbesondere auf Verfahren zur Herstellung des Interferons aus Leukozyten des menschlichen Blutes.

Bekannt ist ein Verfahren zur Herstellung von menschlichem leukozytärem Interferon, das die Leukozytenausscheidung mittels der Ausflokkung der Erythrozyten des ganzen Blutes durch Dextran, die Abtrennung des leukozythaltigen Plasmas, das Ausfällen der Plasmazellfraktion durch Zentrifugieren, die Hamolyse der übriggebliebenen Krythrozyten mit Ammoniumchlorid, das Resuspendieren des Leukozytenniederschlags im Nährmedium, die Biosynthese von Interferon und die Isolierung des Endproduktes vorsieht/siene V. D. Soloviev, G. A. Bektemirov "Interferon v teorii i praktike meditsyny" (Interferon in der medizinischen Theorie und Praxis, M., 1970).

Die Ausbeute an gemäß dem gegebenen Verfahren erhaltenem Endprodukt ist gering und beträgt höchstens $1 . 10^6$ IE pro I des zu verarbeitenden Blutes, was offensichtlich auf die Senkung der Lebensfähigkeit der Leukozyten infolge der Anwendung des gegebenen Verfahrens zur Leukozytenausscheidung zurückzuführen ist. Außerdem ist das nach der Leukozytenabsonderung gebildete Plasma für die weitere Verarbeitung nicht geeignet, weil es Dextran enthält.

Es ist auch ein Verfahren zur Herstellung des menschlichen leukozytären Interferons durch Zentrifugieren des Blutes, die Abtrennung der Leukozyten, die Biosynthese des Interferons und die Isolierung des Endproduktes bekannt (siehe Kauppinen H.—U., Myllylä G. "Human Interferon", 6, 1978, S. 1—13).

Die Ausbeute an gemäß dem gegebenen Verfahren erhaltenem Endprodukt ist höhen, als im obenbeschriebenen Verfahren und beträgt $4 . 10^6$ IE pro des zu verarbeitenden Blutes. Außerdem kann das nach der Abtrennung der Leukozyten erhaltene Plasma für die weitere Verarbeitung oder für die Transfusion bei den Patienten als Blutersatzmittel verwendet werden.

Nichtsbestoweniger ist die Ausbeute an in Übereinstimmung mit dem gegebenen Verfahren erhaltenem Endprodukt nicht hoch, weil die Anzahl der Interferon produzierenden Zellen beim anzuwendenden Verfahren zu deren Ausscheidung durch die Entnahme von buff coat, erhalten nach dem Zentrifugieren des Spenderbluts, höchstens 15% der Anzahl der Leukozyten beträgt, die im Spenderblut enthalten sind.

Aus WO—A—8 301 899 ist ein Verfahren zur Herstellung von humanem Gamma-Interferon durch Abtrennen der Leukozytfraktion (buffy coat) des Blutes, Entfernen der Erythrozyten, Suspendieren der Leukozyten in einem geeigneten Nährmedium, deren Behandlung mit einem mitogenen Mittel und Abtrennen der interferonhaltigen Flüssigkeit von den Zellen bekannt, wobei die Vorbehandlung der Leukozyten mit 200 bis 20.000 IU/ml von α- oder β-Interferon vor der Behandlung mit dem mitogenen Mittel erfolgt.

Offenbarung der Erfindung

Der vorliegenden Erfindung liegt die Aufgabe zugrunde, im Verfahren zur Herstellung von menschlichem leukozytärem Interferon die Ausscheidung der Leukozyten auf die Weise durchzuführen, daß die Ausbeute an Endprodukt erhönt wird.

Die gestellte Aufgabe wird dadurch gelöst, oaß im Verfahren zur Herstellung von menschlichem leukozytärem Interferon, das das Zentrifugieren des Blutes, die Abtrennung der Leukozyten, die Biosynthese der Interferons und die Isolierung des Endproduktes vorsieht, erfindungsgemäß das Zentrifugieren des Blutes bei einer Beschleunigung von 1500 bis 5000 g verwirklicht wird und zue Abtrennung der Leukozyten 15 bis 75% des Volumens des oberen Teils der leukozythaltigen erythrozytären Masse entnommen werden.

In der nach dem Zentrifugieren gebildeten leukozythaltigen erythrozytären Masse sind Leukozyten nach dem Volumen verteilt, und deren Inhalt vermindert sich in einer Volumeneinheit je nach der Entfernung vom buffy coat. Deswegen gestattet das erfindungsgemäße Verfahren zur Leukozytenabtrennung, den abgetrennten Teil der im Spenderblut enthaltenen Leukozyten wesentlich zu erhöhen, was seinerseits gestattet, die Ausbeute an durch Biosynthese erhaltenem Interferon zu steigern.

Das Volumen des entnommenen oberen Teils der leukozythaltigen erythrozytären Masse wird durch den Charakter der Verteilung der Leukozytenkonzentration nach dem Volumen je nach der ausgenutzten Beschleunigung des Zentrifugierens bestimmt.

Bei einer Beschleunigung gegen 1500 g und dem entnommenen Volumen über 75% des oberen Teils der leukozythaltigen erythrozytären Masse steigt stark der Gehalt an Erythrozyten an, die dier Hämolyse ausgesetzt werden, was letzten Endes zur Verminderung der Ausbeute an Endprodukt führt.

Wenn den entnommene Inhalt weniger als 15% des oberen Teils der leukozythaltigen erythrozytären Masse bei einer Beschleunigung gegen 5000 g beträgt, sinkt die Leukozytenausbeute stark, was zur Herabsetzung der Ausbeute an Endprodukt führt.

Beim Zentrifugieren dies Spenderblutes bei einer Beschleunigung unter 1500 g ist es notwendig, die Zeit des Zentrifugierens wesentlich zu steigern, um eine hohe Leukozytenkonzentration im oberen Teil der erythrozytären Masse zu erhalten, was zur Herabsetzung der Ausbeute an Endprodukt führt.

Beim Zentrifugieren des Spenderblutes bei einer Beschleunigung über 5000 g geht die Zerstörung der Leukozyten vor sich, was zur Herabsetzung der Ausbeute an Endprodukt führt.

Beste Ausführungsvariante der Erfindung

Das erfindungsgemäße Verfahren zur Herstellung des menschlichen leukozytären Interferons wird wie folgt durchgeführt.

Das Spenderblut wird in Behälter gesammelt, die einen Stabilisator enthalten. Sofort nach dem Sammeln wird das Blut zentrifugiert, um das Plasma von der erhthrozytären Masse abzutrennen. Nach dem Entfernen des Plasmas werden die oberen 15 bis 75% des Volumens er erythrozytären Masse gesammelt und in einer Ammoniumchloridlösung zur Erythrozytenhämolyse resuspendiert. Das Gemisch wird zentrifugiert und der Leukozytenniederschlag abgetrennt. Der erhaltene Leukozygennierderschlag wird in einem Nährmedium resuspendiert, das das menschliche Blutserum enthält.

Nachher werden die im Nährmedium resuspendierten Leukozyten mit geringen Dosen des menschlichen leukozytären Interferons behandelt. Der erhaltenen Leukozytensuspensionwerden als Induktor ein Virus der Newcastle'schen Krankheit oder ein Sendai-Virus zugesetzt und inkubiert. Nach dem Ablauf der Inkubationszeit gibt man der Suspension ein frisches, das menschliche Blutserum enthaltendes Nährmedium zu und setzt die Inkubation fort. Für die Biosynthese von Interferon kann ein synthetisches Nährmedium beliebiger Zusammensetzung verwendet werden (Eagle MEM, RPMI 1640, McCoy-Nährmedium u.a.), das als eine notwendige Komponente das menschliche Blutserum enthält.

Nach dem Ablauf der Inkubationszeit wird die Kulturflüssigkeit von den Produzentenzellen durch Zentrifugieren abgetrennt. Der Zellniederschlag wird abgetrennt und die Kulturflüssigkeit wird in einem säuren Medium stehengelassen, um das Unduktorvirus zu inaktivieren, danach wird darin der Interferontiter ermittelt.

Nachstehend werden konkrete Beispiele der Verwirklichung der Erfindung angeführt.

### Beispiel 1

Das Spenderblut wurde in Plastiksäcken gesammelt, die einen Stabilisator enthalten. Sofort nach dem Sammeln wurde das Blut bei einer Beschleunigung von 3000 g und einer Temperatur von 4°C zentrifugiert, um das Plasma von der erythrozytären Masse abzutrennen. Nach der Entfernu des Plasmas wurden die oberen 75% der erythrozytären Masse gesammelt und in drei Volumina einer 0,83%igen NH$_4$Cl-Lösung resuspendiert. Das Gemisch wurde bei einer Beschleunigung von 500 g zentrifugiert und der Niederschlag wurde abgetrennt. Der erhaltene Zellenniederschlag wurde wiederum in 10 Volumina einer 0,83%igen NH$_4$Cl-Lösung resuspendiert und bei einer Beschleunigung von 150 g zentrifugiert. Der erhaltene Leukozytenniederschlag wurde im Nährmedium Eagle MEM resuspendiert, das 4% menschliches Blutserum enthielt, um eine Endkonzentration von 10$^7$ Zellen/ml zu erhalten. Die Leukozytenausbeute betrug 5,0·10$^9$ Zellen pro l l Blut oder 80% vom Leukozytengehalt im genzen Blut.

Die im Nährmedium Eagle MEM resuspendierten Leukozyten wurden mit 200 IE/ml menschlichem leukozytärem Interferon bei einer Temperatur von 37°C innerhalb von 2 Stunden behandelt. Nachher wurde der Leukozytensuspension als Induktor ein Virus der Newcastle'schen Krankheit in einer Dosis von 20 hämagglutinierenden Einheiten pro 1 ml zugesetzt und das Gemisch wurde nocht innerhalb von 1 Stunde bei einer Temperatur von 37°C inkubiert. Nach dem Ablauf dieser Zeitspanne wurde das an den Zellen nicht absorbierte Virus durch Zentrifugieren bei einer Beschleunigung von 600 g abgetrennt. Der erhaltene Zellenniederschlag wurde im Nährmedium Eagle MEM in der Endkonzentration von 5,5. 10$^6$ Zellen/ml resuspendiert. Das Nährmedium enthielt 4% menschliches Blutserum. Die Zellensuspension wurde bei einer Temperatur von 37°C unter Rühren innerhalb von 20 Stunden inkubiert.

Nach Abklingen der Inkubierung wurde die Kulturflüssigkeit von den Produzentenzellen durch Zentrifugieren bei einer Beschleunigung von 1500 g abgetrennt. Der Zellniederschlag wurde abgetrennt, und die Kulturflüssigkeit wurde für 3 Tage bei einer Temperatur von 4°C in einem sauren Medium stehengelassen, um das Induktorvirus zu inaktivieren, nachher wurde darin der Interferontiter ermittelt.

Die Ausbeute an Interferon von l l Blut betrug 8.10$^6$ IE.

### Beispiel 2

Das Spenderblut wurde in Plastiksäcken gesammelt, die einen Stabilisator enthielten. Sofort nach dem Sammeln wurde das Blut bei einer Beschleunigung von 2000 g und einer Temperatur von 4°C zentrifugiert, um das Plasma von der erythrozytären Masse abzutrennen. Nach der Entfernung des Plasmas wurden die oberen 75% der erythrozytären Masse gesammelt und in 25 Volumina einer 0,83%igen NH$_4$Cl-Losung resuspendiert. Das Gemisch wurde bei einer Beschleunigung von 250 g zentrifugiert. Der erhaltene Zellenniederschlag wurde in 3 Volumina einer 0,83%igen NH$_4$Cl-Lösung resuspendiert und bei einer Beschleunigung von 150 g zentrifugiert.

Der erhaltene Leukozytenniederschlag wurde im Nährmedium Eagle MEM resuspendiert, das 4% menschliches Blutserum enthielt, um eine Endkonzentration von 10$^7$ Zellen/ml zu erhalten. Die Leukozytenausbeute betrug 5,0.10$^9$ Zellen/pro l l Blut oder 80% von Leukozytengehalt im ganzen Blut.

Die Induktion und die Biosynthese von Interferon wurden ähnlich wie im Beispiel 1 durchgeführt.

Die Ausbeute an Interferon von 1 l Blut betrug 1,2.10$^7$ IE.

### Beispiel 3

Das Spencerblut wurde in Plastiksäcken gesammelt, die einen Stabilisator enthielten. Sofort nach dem Sammeln wurde das Blut bei einer Beschleunigung von 2000 g und einer Temperatur von 4°C zentrifugiert, um das Plasma von der erythrozytären Masse abzutrennen. Nach der Entfernung des Plasmas wurden die oberen 75% der erythrozytären Masse gesammelt und in 50 Volumina einer 0,80%igen NH$_4$Cl-Lösung resuspendiert. Das Gemisch wurde für 10 Minuten bei einer Temperatur von 4°C stehengelassen und bei einer Beschleunigung von 150 g zentrifugiert.

Der erhaltene Zellenniederschlag wurde im Nährmedium Eagle MEM resuspendiert, das 4% menschliches Blutserum in der Endkonzentration von 10$^7$ Zellen/ml enthielt. Di-Leukozytenausbeute betrug 4.10$^9$ Zellen pro I l Blut oder 70% vom Leukozytengehalt im ganzen Blut.

Die Induktion und die Biosynthese von Interferon wurden ähnlich wie in Beispiel 1 durchgeführt.

Die Ausbeute an Interferon von I l Blut betrug 8.10$^6$ IE.

### Beispiel 4

Das Spenderblut wurde in Plastiksäcken gesammelt, die einen Stabilisator enthielten. Sofort nach dem Sammeln wurde das Blut bei einer Beschleunigung von 1500 g und einer Temperatur von 4°C zentrifugiert, um das Plasma von der erythrozytären Masse abzutrennen. Nach der Entfernung des Plasmas wurden die oberen 75% der erythrozytären Masse gesammelt, in 3 Volumina einer 0,84%igen NH$_4$Cl-Lösung resuspendiert und bei einer Beschleunigung von 2500 g zentrifugiert. Der erhaltene Leukozytenniederschlag wurde in 20 Volumina einer 0,84%igen NH$_4$Cl-Lösung resuspendiert und bei einer Beschleunigung von 150 g zentrifugiert.

Der erhaltene Leukozytenniederschlag wurde im Nährmedium MEM resuspendiert, das 4% menschliches Blutserum enthielt, um eine Endkonzentration von 10$^7$ Zellen/ml zu erhalten. Die Leukozytenausbeute betrug 3.10$^9$ Zellen pro I l Blut oder 50% vom Gesamtleukozytengehalt im ganzen Blut.

Die Induktion und die Biosynthese von Interferon wurden ähnlich wie im Beispiel 1 durchgeführt.

Die Ausbeute an Interferon von I l Blut betrug 6.10$^6$ IE.

### Beispiel 5

Das Spenderblut wurde in Plastiksäcken gesammelt, die einen Stabilisator enthielten. Sofort nach dem Sammeln wurde das Blut bei einer Beschleunigung von 5000 g und einer Temperatur von 4°C zentrifugiert, um das Plasma von der erythrozytären Masse abzutrennen. Nach der Entfernung des Plasmas wurden die oberen 15% der erythrozytären Masse gesammelt und in 3 Volumina einer 0,8%igen NH$_4$Cl-Lösung resuspendiert. Das Gemisch wurde bei einer Beschleunigung von 250 g zentrifugiert. Der erhaltene Zellniederschlag wurde in 10 Volumina einer 0,83%igen NH$_4$Cl-Lösung resuspendiert und bei einer Beschleunigung von 150 g zentrifugiert.

Der erhaltene Leukozytenniederschlag wurde im Nahrmedium Eagle MEM resuspendiert, das 4% menschliches Blutserum enthielt, um eine Endkonzentration von 10$^7$ Zellen/ml zu erhalten. Die Leukozytenausbeute betrug 5.10$^9$ Zellen pro 1 Blut oder 80% vom Gesamtleukozytengehalt im ganzen Blut.

Die Induktion und die Biosynthese von Interferon wurden ähnlich wie im Beispiel 1 durchgeführt.

Die Ausbeute an Interferon von I l Blut betrug 6.10$^6$ IE.

### Beispiel 6 (Vergleichsbeispiel)

Das Spenderblut wurde in Plastiksäcken gesammelt, die einen Stabilisator enthielten. Sofort nach dem Sammeln wurde das Blut bei einer Temperatur von 4°C und einer Beschleunigung von 2000 g zentrifugiert, um das Plasma von der erythrozytären Masse abzutrennen. Nach der Entfernung des Plasmas wurde buffi coat gesammelt, in Glasröhrchen übertragen, die einen Stabilisator enthielten. Das Gemisch wurde bei einer Beschleunigung von 160 g zentrifugiert und der Leukozytenniederschlag wurde abgetrennt. Der Niederschlag wurde in 10 ml Nährmedium Eagle MEM resuspendiert, das 10% menschliches Blutserum enthielt. Der erhaltenen Suspension wurde der Stabilisator zugesetzt, und die Suspension wurde mit 8 Volumina einer 0,83%igen NH$_4$Cl-Lösung für die Erythrozytenhämolyse vermischt. Nach einigen Minuten wurde das Gemisch bei einer Beschleunigung von 160 g zentrifugiert.

Der erhaltene Leukozytenniederschlag wurde im frischen Nährmedium Eagle MEM bis zur Endkonzentration von 10$^7$ Zellen/ml resuspendiert. Die Endausbeute an Leukozyten betrug 0,9.10$^9$ Zellen pro I l Blut oder 15% vom Leukozytengehalt im Spenderblut.

Die Induktion und die Biosynthese von Interferon wurden ähnlich wie im Beispiel 1 durchgeführt.

Die Ausbeute an Interferon von I l Blut betrug 4.10$^6$ IE.

Somit gestattet das erfindungsgemäße Verfahren, die Interferon-Ausbeute wesentlich zu erhöhen.

### Industrielle Anwendbarkeit

Die vorliegende Erfindung kann am erfolgreichsten in der pharmazeutischen Industrie Verwendung finden.

### Patentanspruch

Verfahren zur Herstellung von menschlichem leukozytärem Interferon, das das Zentrifugieren des Blutes, die Abtrennung der Leukozyten, die Biosynthese des Interferons und die Isolierung des Endproduktes vorsieht, dadurch gekennzeichnet, daß das Zentrifugieren des Blutes bei einer Beschleunigung von 1500 bis 5000 g durchgeführt wird und zur Abtrennung der Leukozyten 15 bis 75% des Volumens des oberen Teils der leukozythaltigen erythrozytären Masse entnommen werden.

**Revendication**

Procédé de production d'interferon leucocytaire humain qui prévoit a centrifugation du sang, la séparation des leucocytes, la biosynthèse de l'interferon et l'isolement de produit final, caractérisé en ce que la centrifugation du sang est accomplie à une accélération de 1500 à 5000 g et on prélève, pour la séparation des leucocytes, 15 à 75% du volume de la partie supérieure de la masse érythrocytaire contenant des leucocytes.

**Claim**

A process for the manufacture of human, leucocyte interferon, providing centrifugation of the blood, separation of the leucocytes, biosynthesis of the interferon and isolation of the end product, characterized in that the centrifugation of the blood is carried out at an acceleration of 1500 to 5000 g and that for separating the leucocytes 15 to 75% of the volume of the upper part of the leucocyte-containing, erythrocyte mass is removed.